Europäisches Patentamt

European Patent Office

Office européen des brevets

(11) Publication number: **0 223 153**
A2

# (12) EUROPEAN PATENT APPLICATION

(21) Application number: **86115334.4**

(22) Date of filing: **05.11.86**

(51) Int. Cl.⁴: **C 07 K 15/22,** C 07 K 3/08, A 61 K 37/14

(30) Priority: **06.11.85 IT 2273085**

(43) Date of publication of application: **27.05.87 Bulletin 87/22**

(84) Designated Contracting States: **AT BE CH DE ES FR GB GR IT LI LU NL SE**

(71) Applicant: **MEDIOLANUM FARMACEUTICI Srl, Via S. Giuseppe Cottolengo 31, I-20100 Milano (IT)**

(72) Inventor: **De Ambrosi, Luigi, Via Caroucci 8, Santhia Alessandria (IT)**
Inventor: **Pagella, Piergiuseppe, Fraz. Capraglia 56, Isola S. Antonio Alessandria (IT)**

(74) Representative: **Gervasi, Gemma, Dr., NOTARBARTOLO & GERVASI Srl 33, Viale Bianca Maria, I-21100 Milano (IT)**

(54) **Iron derivatives of globin and of acetylglobin endowed with high bioavailability process for their preparation and pharmaceutical formulations.**

(57) Iron derivatives of globin and of acetylglobin endowed with high bioavailability, containing quantity of iron changing from 2 to 20% in weight, are useful pharmaceutical compositions to treat iron deficency anemia.

The process for obtaining the above mentioned iron derivatives has been carried out through the following phases;

a) Preparation of globin starting from red blood cells of animals;

b) Preparation of acetylglobin through the acetylation of globin;

c) Preparation of iron glucidic dervatives;

d) Preparation of iron acetylglobin and of iron globin through the reaction respectively of acetylglobin and globin with iron-glucidic derivatives or with inorganic iron salts.

The products thus obtained have an high solubility at neutral and alkaline pH and, orally administered, increase the basal iron levels in the serum without damaging the gastrointestinal system.

-1-

"Iron Derivatives of Globin and of Acetylglobin endowed with high bioavailability, process for their preparation and pharmaceutical formulations"

The present invention refers to a new class of iron- derivatives obtained by the reaction of Globin or Acetylglobin, extracted  by red blood cells of animals, with organic or inorganic iron compounds.

These proteic iron-derivatives contain an high percentage of complex bioavailable iron and are very useful for preventive treatments and to treat iron deficency anemia.

In the human body iron, which is very important for various biological processes, is found in small quantities controlled by mechanisms which are capable of avoiding phenomena such as deficency or accumulation.

The phisiological loss of iron, caused by epitelial desquamation, biliar excration, gastrointestinal or mestrual blood loss are offset by mechanisms of intestinal assimilation, whereas particular situations such as pregnancy, breast-feeding, infantile growing, inadeguate diet, syndroms of bad processes of absorption, are very often lincked with iron deficency anemia which is usually treated by oral administration of iron salts, which often cause phenomena of gastroenterical intolerance.

A remarkable process has been achieved with the use of Ferritine, iron globulin formed by a proteic part, apoferritine, which surraunds a "core" of Iron corresponding to 20% as far as the dryed weight is concerned.

Ferritine does not damage the gastroenteric apparatus but, because of the reduced availability of sources from which it is extracted (the spleen of the horse) it is very expensive and consequently it is little spread. Nevertheless the results obtained by the use of Ferritine show that the optimal carrier of iron, in order to carry out oral therapy which does not cause damaging side-effects to the gastroenteric system, has a proteic nature.

Proteins of animal and vegetable nature with or without any alterations (albumin, casein, soya proteins, etc.).

Nevertheless the interaction between these proteins and iron salts forms not homogeneus, instable, less soluble, and with a low content of iron complexes.

The present inventione refers to a new class of proteic iron derivatives which have a high content of bioavailable iron, which are stable and very soluble at a neutral and alkalin pH and consequently very soluble intestine.

These after being orally administered to the animals for experiment are capable of increasing the values of sideremia and they do not cause side-effects to the gastroenteric system. In addition the iron assimilation of the iron-derivatives, which are the object of the present invention, follows the normal absorption pathways through physiological checking mechanisms which regulate the intestinal assimilation of iron.

Iron-derivatives of Globin and Acetylglobin, obtained from red blood cells of animals, are endowed with quantities of iron varying from 2 to 20% in weight, and are useful for the preparation of pharmaceutical composition suitable to treat iron deficency anemia in man, are the object of the present invention.

The object of the present invention relates also to the processes for the preparation of said iron-derivatives which are carried out by the following phases:

a) Preparation of Globin starting from red blood cells of animals;

b) Preparation of Acetylglobin from Globin;

c) Preparation of iron glucidic-derivatives;

d) Preparation of iron Acetylglobin and of iron globin through the reaction rispectively of Acetylglobin and Globin with iron glucidic-derivatives.

As an alternative to the reaction with iron glucidic-derivatives, Globin and Acetylglobin are made react with inorganic iron salts. According to the present invention these and others characteristics of the products and of the process will be mainly pointed out by the following description which refers to better modalities being used to carry out the present invention and which is reported to the purpose of illustrating but not limitating.

For the preparation of Globin and Acetylglobina (phase A) animal blood, which can have bovine, ovine, swine origin, which is treated with sodium citrate (4 g/liter), is used.

The corpuscolar mass is washed with saline solution dissolved in acetone and added to a solution of HCl conc. in acetone, being the ratio in weight between animal blood and HCl 1:0,002.

Globin is allowed to precipitate at a temperature of 3-10° C, it is centrifugated, submitted to anhydrification, pulverized and purified by dissolving it in distilled water at a temperature of 35-45°C, filtered and ultrafiltered, and the purified Globin is regained at a concentrated liquid state (solution at 2,5 % in weight).

For the preparation of Acetylglobin (phase b), the Globin obtained in phase a) is brought to a pH 9,5-10,5 by adding a solution of NaOH N/10. The acetic anhydride and at the same time a solution of NaOH N/10 are slowly added so that to keep the pH at values varying from 7,5 to 8,5. The mixture is filtered and acidified with a HCl 5N solution until pH 3-3,5.

The precipitate thus obtained is separated by filtration, suspended in distillate water and dissolved again adding NaOH until it reahes a pH 7,5-8,5. The react mixture thus obtained is filtred and the processed of precipitation, filtration, dissolution and clearin are repeated and the purificated mixture is, at last, filtered or dialyzed and lyophilizated. The lyophilizate is formed by acetylate globin in which the level of acetylation changes according to the quantity of acetic anhydride being used, and it varies, preferably, from 10 to 100%.

For the preparation of iron glucidic-derivatives (phase c) glucidic mixture and iron chloride are mixed.

A process of molecular condensation with a gel solvatated structure having characteristics of stability and solubility is started through changes of pH.

Iron glucidic-derivatives used in the present invention, are preferably saccarate and fructate.

Phase d) is carried out by different alternative modalities according to the kind of product you want to prepare, iron Acetylglobin or iron Globin, and according to the kind of iron reagent used.

For the preparation of iron Acetylglobin by making react Acetylglobin with iron glucidic-derivative, Acetylglobin, prepared as in phase b), is dissolved in distillate water and slowly added, under refluxing conditions, at room-temperature, with a solution of iron glucidic-derivative prepared as in phase c). The ratio, in weight, between Acetylglobin and iron varies from 19:1 to 4:1.

Acetylglobin solution has preferably a concentration which varies from 2,5 % to 10%, while iron glucidic-derivative has preferably a concentration which varies from 5% to 10%.

The mixture is kept stirred for 1-3 hours and then is slowly treated with HCl 0,1 N until the value of pH is reduced from 7 to 5, the precipitate being thus obtained.

The precipitate is separated by filtration or centrifugation, washed with a diluite solution of HCl, sospended in distillate water and dissolved again by slowly adding a solution of NaOH 1N until pH 7.

The clear mixture is dialyzed or ultrafiltered and, at last, lyophilizated.

The product being thus formed, made of iron acetylglobin, has: a content of complex iron which can vary from 5 to 20% in weight, a content of proteic Nitrogen which can vary from 15,6 to 12,8 % in weight and a grade of solubility in water equal to 30% p/v.

As an alternative to the process already described, the Acetylglobin solution is made react with a solution of an inorganic iron salt, preferably $FeCl_3 \cdot 6H_2O$, with a ratio in weight between Acetylglobin and iron going from 49:1 to 15:1, at a room-temperature, kept under refluxing condition for 10-60 minutes. The solution of inorganic iron salt has, preferably, a concentration which can vary from 2,5 to 10%. A precipitate is formed and it is treated as described above.

The final product, obtained by lyophilisation, is made of iron acetylglobin and has a content complex iron varying from 2 to 6% in weight and a content of proteic Nitrogen varying from 15 to 16% in weight and a level of solubility in water equal to 30% p/v.

Acting according to the described process, the iron Globin complex is preparated too making react a solution of Globin coming from phase a) with a solution of iron glucidic-derivatives, in a Globin/iron ratio varying from 19:1 to 4:1 or with a solution of $FeCl_3 \cdot 6H_2O$ in a Globin/iron ratio varying from 49:1 to 15: 1, keeping it stirred for 30-60 min.

The concentration of the globin solution varies, preferabily, from 2,5 to 10%; the concentration of iron glucidic-derivative solution varies, preferably, from 5 to 10% and the concentration of iron salt solution varies, preferably, from 8 to 12%.

During the treating of Globin with iron glucidic-derivatives, an iron glucidic complex with a quantity of complexed iron, which can vary from 5 to 20% in weight, a content of proteic Nitrogen, which can vary from 13 to 15% in weight and a level of solubility in water corresponding to 20% p/v are obtained.

During the treating of Globin with $FeCl_3 \cdot H_2O$, an iron globin complex with a content of complexed iron, which can vary from 2 to 6% in weight, a content of proteic Nitrogen, which can vary from 15 to 16% in weight and a level of solubility in water corresponding to 20% p/v are obtained.

The compounds being thus obtained by the teating with $FeCl_3 \cdot 6H_2O$ have a content of iron sensibly lower than the compounds obtained by the treating with glucidic iron-derivatives, nevertheless each of the two kinds of products finds its specific therapeutical use. The iron contained in the products of the present invention is complitely complexed: the lack of not binding iron is verified by the precipitation with ammonium sulphate and with HCl N/10 and it is shown by the compete solubility in an alkaline milieu, a condition in which the iron ion precipitates.

The proof of precipitation with ammonium sulphate is carried out by adding to a solution at 10% of quantity of iron-derivate taken into exame, 30% (p/v) of ammonium sulphate.

The precipitate being thus obtained is exactly alike to the previous iron-derivative, which is again soluble in water, whereas there is no free iron in the solution. The compounds obtained, as described above, are stable in acid milieu and they have a good solubility in water and in alkalin milieu, that is to say at values of pH recognizable in the intestine where complex iron is quickly released after having being orally administered, increasing the values of the basal sideremia without causing any damage to the gastrointestinal system. The present invention refers to pharmaceutical formulations (vial, tablets, capsules, syrups, small envelopes with granulate etc.), which contain determinate quantity of iron globin and acetylglobin derivatives obtained from red blood cells, useful to treat and to prevent the iron deficency anemia.

The following formulation are mentioned just as an example which does not have to be considered limiting, as far as the present invention is concerned:

- Vials containing 10-20-40-100 mg of iron in the shape of iron derivatives of proteins or of acetylprotein from red blood cells and besides watery solvent, flavourings, stabilizing, etc. usually used in pharmaceutical technics.

- Tablets containing 10-20-40-100 mg of iron in the shape of iron

derivatives of proteins or acetylproteins from red blood cells, and besides excipients, disgregating elements, etc., usually used in pharmaceutical technics.

- Capsules containing 10-20-40-100 mg of iron in the shape of iron-derivatives of proteins or acetylproteins from red blood cells.

- Small monodoses envelopes containing a granulate with 10 20 40 100 mg of iron in the shape of iron-derivatives of proteins or acetylproteins. from red blood cells.

- Syrups containing 1 2 4 10 mg/ml of iron in the shape of iron-derivatives of proteins or acetylproteins from red blood cells, and besides watery solvent, flauvorings, stabilizings, etc., usually used in pharmaceutical technics.

TOXICITY

The acute toxicity has been valued in the mice after an orally administration of iron-derivatives according to the invention, and it has been found a DL50 that is in any case higher than 4000 mg/Kg.

On the contrary the ferrous sulphate, after an oral administration has in mice a DL50 which corresponds to 1500 mg/Kg.

Sideremia after oral administration

The capacity of some of iron derivatives, which are mentioned in the present invention to increase the basal values of sideremia, has been valued in 180-200 g rats S.D. which are fasting since 18 hours. The products have been administered by oral gavage two hours before the sacrifice of animals, with doses containing a quantity of iron equal to 2 mg/Kg.

The dermination of the quantity of iron in serum has been carried out by a spectrophotometer with a batophenantroline method (colorimetric method, Beohringer Mannheim). The results are reported in table I.

TABLE I

| Treatment | Iron in the serum μg/100 ml |
|---|---|
| Saline | 150 ± 20.4 |
| Example I | 322 ± n6.4 |

0223153

| | |
|---|---|
| Example 4 | $288 \pm 18.0$ |
| Example 5 | $323 \pm 19.2$ |
| Ferrous soulphate | $269 \pm 36.7$ |

The following examples are reported to the purpose of illustrating, not limiting the invention.

EXAMPLE 1

a) Globin preparation starting from red blood cells

500 ml of bovino citrate blood are centrifugated at 3000 rpm. The corpuscolar massa, washed twice in a volume of 300 ml of saline solution is dissolved in a volume of 800 ml of acetone at 5°C and a volume of 100 ml of a solution formed with HCl conc. and acetone (1:10) is added. The proteic suspension thus obtained, is kept at a low temperature (5°C) for 12 hours making easier the globin precipitation. The precipitate is separated by centrifugation, anidryficated and polverized.

An amount of 60 g of globin, which are soon after purified by dissolution in 5 liters of water at 40°C followed by a filtration through paper till it becomes clear and then by a ultrafiltration at 10000 of cut off, are obtained.

An amount of 50 g of globin purified in solution at 2.5 % are obtained.

b) Preparation of acetilglobine

An amount of 400 ml of the compound a) (10g of globin) are slowly made alkaline with NaOH 1 N to pH 10 under stirring. Then 5 ml of acetic anhidride and at the same time NaOH, to keep the Mixture at a pH of about 8, are added. When the addition is onded the reaction mixture is kept stirring for 60 min. at a room temperature.

The opalescent is filtered off untill it becomes clear, then it is slowly made acid with HCl 5 N till pH 3-3.5.

The precipitate being formed is filtered off, suspended in a volume of 100 ml of $H_2O$ and added with NaOH till a complete dissolution (pH about 8). The mixture is filtered off again and then made acid with HCl till pH 3+3.5.

The precipitate being formed is filtered off, suspended in a volume of 100 ml of $H_2O$ and dissolved again by the addition of NaOH to pH 8.

The clear mixture is dialyzed or ultrafiltrated and then Lyophilized.

The lyophilizate obtained is formed by an amount of 7 g of globine acetylated, whose level of acetilation corresponds to 95%.

The acetylation level is given by the ration between the percentage of acetylate groups in comparison with the groups which are not yet acetylated of the previous globin, and it is found by the ninidrine reaction of the free amminic groups (J. Biol. Chem. 211,1954,907).

c) Preparation of the saccarated iron and fructate iron.

In the preparation of fructate-iron an amount of 50 g of esaidrate chloride iron are dissolved in 2 Liters of distilled water and put slowly in a volume of 500 ml of a mixture containing 300 g of fructose.

After having mixed the compounds the pH is slowly moved till 7.8-8.5 with a mixture of KOH at 20%. It is kept under stirring for many hours, kipping the pH towards values of 8-8.5.

When the compound is stable it is filtered. It is dissolved 1: 100 and ultrafiltered at 1000 cut of with continual addition of distilled water till negative reaction of the ferric iron the permeated. It is concentrated till about 800. The compound, after having been filtered is liophilize. Yeld=80 g of powder at strenght in iron = 10%.

In the preparation of saccarated-iron an amount of 50 g of esaidrate chloride iron are dessolved in 2 liters of distilled water and are slowly poured in 1 liter of a solution containing 500 g of saccarose.

After having mixed it, the pH is moved slowly till 8.0+8.5 with a mixture of metilglucamine at 20%. It is kept under stirring for many hours taking the pH back at its previous values with other addition of metilglucamine. When compound is stable it is heated at 50°C for about 30 min and it is filtered. The mixture is dissolved to 5 liters and it is ultrafiltered at a cut off of 1000 with continuous addition of distilled water till negative reaction of ferric iron in the permeated. It is concentrated to 500 ml, it is filtered and

0223153

lyophilized. Yeld: 50 g of powder with a iron strengh of 10%.

d) Preparation of the iron-acetylflobine complex with the use of the saccarated iron.

An amount of 3 g of acetylate globine, obtained as described above in phase b, are dissolved in 60 ml of $H_2O$ (pH about 7.5), and are slowly added under stirring with an amount of 6 g of saccarated iron (iron: 10%) prepared as described in phase c), which are dissolved in 6 ml of $H_2O$. When the addition is ended the reaction is allowed to proceed for 2 hours under refluxing condition at room temperature.

The obtained mixture is slowly acidified with HCl 0.1 N till it precipitates to pH 5.

The precipitate being formed is filtrated off, it is washed with 30 ml of HCl 0.01 N, it is suspended in 30 ml of $H_2O$ and it is dissolved again by adding slowly NaOH till pH 7.

The clear mixture is then ultrafiltered of dyalized and lyophilized. The solid obtained by the lyophilization (2.7 g) has a content of complex iron corresponding to 14.1 %, a level of solubility in water corresponding to 30% and percentage of protein nitrogen corresponding to 12.8 %.

EXAMPLE 2

Preparation of iron acetylglobine complex by the use of iron-fructate.

With the same modalities described in the first example a complex iron is obtained starting from an amount of 3 g of acetylated globin and from an amount of 6 g of fructate iron (iron=10%) prepared as described in phase c of the fist example.

The solid obtained by the lyophylization has content of complex iron corresponding to 12.4 %, a level of solubility in water corresponding to 40% and a percentage of proteic nitrogen corresponding to 14%.

EXAMPLE 3

Preparation of iron acetylglobine complex with the use of iron chloride.

An amount of 1 g of acetylglobine, obtained as described in phase b of the first example, is dissolved in 10 ml of water (pH 7) and added to 0.83 g of iron chloride dissolved in 10 ml of $H_2O$ (pH 2). The reaction is kept stirred for about 30 min. The precipitate being formed is filtrated off, washed with 30 ml HCl 0.01 N and dissolved again by adding slowly NaOH till pH 7.5. The mixture is filtered off, dyalized and lyophilized. The solid obtained by lyophilization has a content of complex iron corresponding to 4.5%, a level of solubility corresponding to 30% and a percentage of protein nitrogen corresponding to 15%.

EXAMPLE 4

Preparation of iron globin complex with the use of fructate iron.

An amount of 5 g of globin, obtained according to phase a of the example I, are dissolved in 200 ml of water (pH 3.5) and added with an amount of 10 g of fructate iron (iron: 10%) compounded as described in phase c) of the first example, dissolved in 100 ml of $H_2O$. The reaction is allowed to proceed under refluxing condition for about 30 min. A precipitate is formed and it is collected by centrifugation, washed with distilled water and completely dissolved with NaOH till pH 7.5. The mixture is filtered off, dyalized and lyophilized. The solid obtained by lyophilization (4.5 g) has a content of complex iron corresponding to 12.2 % and a percentage of proteic nitrogen corresponding to 14%.

EXAMPLE 5

Preparation of iron globin complex with the use of saccarated iron.

According to the same modalities described in the example 4, an iron complex is formed starting from an amount of 5 g of globin obtained according to phase a) of the first example and an amount of 10 g of saccarated iron (iron:10%) compounded as described in phase c) of the first example.

The solid obtained by lyophilization has a content of complex iron corresponding to 12.5 % and a percentage of protein nitrogen corresponding to 14%.

EXAMPLE 6

Preparation of iron globin complex with the use of iron chloride.

According to the same modalities described in the example 4, a complex iron is obtained starting from an amount of 1 g of globin obtained according to phase a) of the first example and 0.83 g of iron chloride.

The solid obtained by lyophilization has a content of complex iron corresponding to 5% and a percentage of proteic nitrogen corresponding to 15.2.

0223153

CLAIMS

1. Iron-derivative of Globin and Acetylglobin endowed with high bioavailability, containing quantity of iron changing from 2 to 20% in weight, useful in the treatment of iron deficiency anemia.

2. Process for the preparation of iron-derivatives of globin and acetylglobin endowed with high bioavailability, containing quantity of iron changingfrom 2 to 20% in weight useful in the treatment of iron deficiency anemia in the man and characterized by the following phases:

a) Preparation of globine starting from red blood cells of animals;

b) Preparation of acetylglobine through the acetylation of the globine;

c) Preparation of iron glucidic. derivatives;

d) Preparation of iron acetylglobine and of iron globine through the reaction respectively of acetylglobine and globine with iron compounds.

3. Process according to claim 2, characterized by the fact that the said preparation of globine is carried out treating animal blood, submitted to centrifugation, with a solution of HCl concentrated, being the ratio in weight between animal blood and HCl (chloridric acid) of 1:0.002, at a temperature varying from 3° to 10°C.

4. Process according to claim 2, characterized by the fact that the said compound of acetylglobine is carried out by adding slowly acetic anhydride to a watery solution of globine keeping the pH at values varying from 7.5 to 8.5 by adding at the same time a solution of sodium hydrate.

5. Process according to claim 2, characterized by the fact that the said compound of glucidic iron derivatives is carried out by a process of molecular condensation with a gel selvatate structure which passes through a passage of purification and concentration with technologies of ultrafiltration.

6. Process according to claim 2, characterized by the fact that the said preparation of the iron acetylglobine is carried out by slowly adding, at a room temperature, a solution of this glucidic iron derivative at a cocnentration varying from 5% to 10% to a solution of

- 14 -

0223153

the said acetylglobine varying from 2.5 % to 10%, the reaction mixture is kept stirred for 1-3 hours, than the ironacetylglobine is made precipitate with HCl till it reaches a pH which varyes from 4 to 5.

7. Process according to claim 6, characterized by the fact that the ratio between acetylglobine and iron used to prepare the iron acetylglobine varyes from 19 : 1 to 4 : 1.

8. Process according to claim 6, characterized by the fact that the precipitated iron acetylglobine is separated by the solution, than it is washed with a HCl diluite mixture, dissolved again in a NaOH mixture and the obtained solution  is filtered or dialized or lyophilized.

9. Process according to claim 2, characterized by the fact that the said preparation of iron acetylglobine is carried  out adding at a room temperature a mixture of inorganic iron salt at a concentration which varyes from 8% to 12% to a mixture of acetylglobine at a concentration which varyes from 2.5 % to 10% and the reaction is allowed to proceed for 10-60 minutes under refluxing condition.

10. Process according to claim 9, characterized by the fact that the ration between acetylglobine and iron used to prepare iron acetylglobine varyes from 49 : 1 to  15 : 1.

11. Process according to claim 2, characterized by the fact that said compound of iron acetylglobine is carried out adding at a room temperature a mixture of said glucidic iron derivative at a concentration which varyes from 5 to 10%  to a mixture of globine, at concentration which varyes from 2.5% to 10%, the reaction mixture is kept stirred for 30-60 minutes.

12. Process according to claim II, characterized by the fact that the ratio between globine and iron used to prepare ironglobine varyes from 19 : 1 to 14 : 1.

13. Process according to claim 2, characterized by the fact that the said compound of iron globine is carried out adding at a room temperature a mixture of iron salt at a concentration which varyes from 2.5% to 10% to a mixture of globine at a concentration which varyes from 2.5 % to 10%. The reaction mixture is kept stirred for 30-60 minutes.

14. Process according to claim 13, characterized by the fact that the ratio between globine and iron used to prepare iron globine varyes from 49 : 1 to 15 : 1.

15. Pharmaceutical compounds useful to treat iron deficiency anemia of man which contain as active principle iron derivatives of globine and acetylglobine as it has been shown in the claims from 1 to 14.